# EUROPEAN PATENT APPLICATION

(11) **EP 0 916 350 A1**
(43) Date of publication of application: **19.05.1999**
(21) Application number: 98308507.7
(22) Date of filing: 16.10.1998
(51) Int. Cl.: A61L 2/24, A61L 2/26, G01M 3/32, G01M 3/28, B01J 3/04, B01J 3/00

(54) **Autoclaves**

(30) Priority: 08.11.1997 GB 9723595
(71) Applicant: Smiths Industries Public Limited Company, London, NW11 8DS (GB)
(72) Inventor: Hannant, Keith, Rustington, West Sussex BN16 2QN (GB)
(74) Representative: Flint, Jonathan McNeill

(57) **Abstract**

An autoclave control unit (4) tests operation of the autoclave by turning on a pump (10) to evacuate the pressure vessel (1) and then monitors rise in pressure within the vessel to detect any leaks. The control unit (4) then switches a solenoid valve (14) connected to a needle valve (13) so that a predetermined leak is connected to the vessel (1). The control unit (4) monitors the rise in pressure to determine if this is within predetermined limits and, if it is, it then monitors the output of an air detection system (23) to check if this detects the predetermined leak. The results of the tests are printed by a printer (31).

## Description

This invention relates to autoclaves of the kind including a pressure vessel, a pump for evacuating the pressure vessel, a pressure sensor for monitoring pressure within the pressure vessel, and a control unit.

Autoclaves, such as autoclave sterilizers, must be tested frequently to ensure safe operation and to check that objects in the autoclave are subject to the desired treatment cycle. One of the tests involves testing for leaks in the pressure vessels and testing that the autoclave's air detection system is functioning correctly. These tests are carried out by an engineer who evacuates the pressure vessel, using the autoclave vacuum pump, with all connections to the chamber sealed off. The engineer monitors the rise in pressure of the chamber as air enters through any leaks. If the rate of pressure rise is too high, the autoclave fails the test and is returned for servicing. If the autoclave passes this test, the engineer connects a calibrated leak to a port opening into the chamber, while it is still at reduced pressure, and checks if the autoclave's air detection system responds to the leak within a set time. The autoclave passes the test if the air detection system does detect the leak.

Although this testing system is reliable, it requires the presence of a trained engineer and, therefore, adds to the cost of using the autoclave.

It is an object of the present invention to provide an improved autoclave.

According to the present invention there is provided an autoclave of the above-specified kind, characterised in that the control unit is operable first to cause the pump to evacuate the vessel, then to seal the vessel, to monitor an output of the pressure sensor and to determine whether the rate of rise of pressure is within predetermined limits so as thereby to check for leaks into the vessel, and that the control unit is operable to provide a signal indicative of whether leakage into the vessel is within predetermined limits

The autoclave preferably includes a predetermined leak and a valve for selectively connecting or isolating the leak from the pressure vessel, the control unit being arranged to connect the leak to the vessel after the vessel has been evacuated by the pump and to monitor the output from the pressure sensor to determine whether the rate of rise of pressure is within second predetermined limits, the control unit being arranged to provide a signal indicative of whether the predetermined leak is within the second predetermined limits. The predetermined leak may be a needle valve, the valve for selectively connecting or isolating the predetermined leak from the pressure vessel being a solenoid valve, and the control unit being connected with the solenoid valve to control operation thereof. The autoclave preferably includes an air detection system, the control unit being operable to open the valve connecting the predetermined leak to the vessel and to monitor an output from the air detection system to check whether the air detection system detects the predetermined leak, and the control unit being arranged to provide a signal indicative of whether the air detection system is operating correctly. The control unit preferably only monitors the output of the air detection system if the predetermined leak is within the predetermined limits. The autoclave may include a printer, the signals from the control unit being supplied to the printer to provide a printed representation of the autoclave function. The control unit may be arranged to disable operation of the autoclave if the control unit detects a fault in the autoclave.

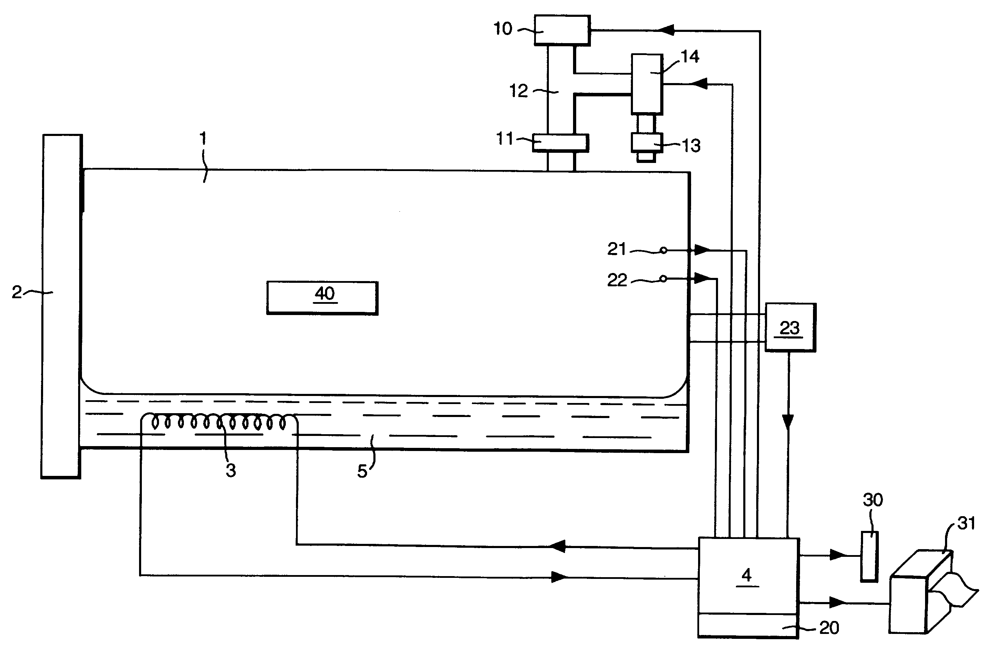

An autoclave sterilizer according to the present invention, will now be described, by way of example, with reference to the accompanying drawing, which shows the sterilizer schematically.

The sterilizer has a pressure vessel or chamber 1 with a door 2 through which articles to be sterilized are placed in the chamber. An electrical heating coil 3 at the bottom of the chamber 1 is energized by a control unit 4 to raise the temperature of water 5 and produce steam. When the chamber 1 is sealed, the steam raises the pressure and temperature in the chamber sufficiently for the articles to be sterilized.

The sterilizer also has a vacuum pump 10 connected via a filter 11 and a pipe 12, which opens into the upper end of the chamber 1. Operation of the vacuum pump 10 is controlled by the control unit 4. When the pump 10 is off, it prevents passage of air through the pump. A needle valve 13 with a predetermined, calibrated leak rate is also connected to the pipe 12 at a location between the pump 10 and the filter 11, via a normally-closed solenoid valve 14. The solenoid valve 14 is also connected to the control unit 4.

The control unit 4 includes a timer 20 and receives outputs from various sensors, such as a pressure sensor 21, a temperature sensor 22 and an air detector system 23. The air detector system 23 monitors variations in pressure and temperature with time. In an effective air removal cycle, these values will have a particular process profile. If the parameters are outside particular ranges, this can indicate that air removal has not been effective, so the cycle is aborted. In particular, a minimum vacuum level has to be achieved within a particular time. If this is achieved, the chamber 1 is sealed and pressure is monitored to detect leakage into the chamber. The water 5 is then heated under vacuum and a second vacuum phase is performed, pressure being monitored to ensure a particular vacuum level is achieved in a particular time. Subsequently, pressure is monitored at a set temperature to determine if there is good correlation with steam table values. Outputs from the control unit 4 are connected to a display panel 30 and a printer 31.

In normal operation, the user places articles 40 to be sterilized, which may be porous, in the sterilizer chamber 1 and closes the door 2. When the user starts the sterilization cycle, the control unit 4 starts the pump 10 so that it evacuates air in the chamber 1, and then stops the pump to seal the chamber before the heating coil 3 is energized. The sterilizer completes the sterilization cycle in the usual way.

The control unit 4 is arranged automatically to perform three different test procedures, as follows.

The first test is a leak test. To do this, the control unit 4 turns on the vacuum pump 10 with the solenoid valve 14 and any other connections to the chamber 1 closed. The pump 10 pulls a vacuum in the chamber 1 of about 12kPa absolute. The control unit 4 then monitors the output of the pressure sensor 21 for a pre-set time, between about 2 to 5 minutes, to monitor the rate of leakage into the chamber 1. If the leakage is less than 2kPa/min, the control unit 4 determines that the leak rate is satisfactory and causes the printer 31 to print out a message to indicate a satisfactory result. If the leakage rate is too high, a message is printed indicating this. A warning message may also be displayed on the display panel 30. The control unit 4 could also disable the sterilizer, preventing further sterilization cycles being started until the leak is corrected.

The next test is to test correct operation of the needle valve 13. While the chamber 1 is still at a reduced pressure, the control unit 4 opens the solenoid valve 14 so that air can leak into the chamber via the needle valve 13. The control unit 4 monitors the pressure output from the sensor 21 and thereby monitors the rate of rise of pressure within the chamber 1. The control unit 4 compares this rate of rise against a predetermined range of rates of rise of pressure to determine whether the rate of rise is within the required limits. If the rate of rise is too fast, it indicates that the needle valve 13 is open too far; if the rate of rise is too slow, it indicates that the needle valve is not open sufficiently, or that there is some blockage in the leak path restricting flow.

If the leak rate is outside the prescribed limits, the control unit 4 signals the printer 31 to print an appropriate message advising the user to have the needle valve 13 checked. A warning message may also be displayed on the display panel 30. The control unit 4 could also disable the sterilizer, preventing further sterilization cycles being started until the leak rate of the needle valve 13 is corrected.

If the leak rate is within the prescribed limits, the control unit 4 continues with an air detector function test, to test correct operation of the air detection system 23. In this, the control unit 4 causes the sterilizer to run a standard sterilization cycle and then introduces a small, controlled leak, by opening the solenoid 14 so that air can flow through the, now calibrated and verified, needle valve 13 during a phase when the chamber 1 is below atmospheric pressure. If the air detection system 23 detects the entry of air into the chamber 1, the control unit 40 determines that the sterilizer is functioning satisfactorily and the printer 31 prints out an appropriate message. If no leak is detected, a fault is indicated on the printout.

Because the leak rate of the needle valve 13 is automatically checked in this way, there is no risk of the air detection test being run with an incorrect leak rate. This avoids the need for the presence of an engineer to check correct operation of the air detection system 23.

## Claims

1. An autoclave including a pressure vessel (1), a pump (10) for evacuating the pressure vessel, a pressure sensor (21) for monitoring pressure within the pressure vessel (1), and a control unit (4), characterised in that the control unit (4) is operable first to cause the pump (10) to evacuate the vessel (1), then to seal the vessel, to monitor an output of the pressure sensor (21) and to determine whether the rate of rise of pressure is within predetermined limits so as thereby to check for leaks into the vessel (1), and that the control unit (4) is operable to provide a signal indicative of whether leakage into the vessel (1) is within predetermined limits.

2. An autoclave according to Claim 1, characterised in that the autoclave includes a predetermined leak (13) and a valve (14) for selectively connecting or isolating the leak from the pressure vessel (1), that the control unit (4) is arranged to connect the leak (13) to the vessel (1) after the vessel has been evacuated by the pump (10) and to monitor the output from the pressure sensor (20) to determine whether the rate of rise of pressure is within second predetermined limits, and that the control unit (4) is arranged to provide a signal indicative of whether the predetermined leak (13) is within the second predetermined limits.

3. An autoclave according to Claim 2, characterised in that the predetermined leak is a needle valve (13), that the valve for selectively connecting or isolating the predetermined leak from the pressure vessel is a solenoid valve (14), and that the control unit (4) is connected with the solenoid valve (14) to control operation thereof.

4. An autoclave according to Claim 2 or 3 including an air detection system (23), characterised in that the control unit (4) is operable to open the valve (14) connecting the predetermined leak (13) to the vessel (1) and to monitor an output from the air detection system (23) to check whether the air detection system detects the predetermined leak, and that the control unit (4) is arranged to provide a signal indicative of whether the air detection system (23) is operating correctly.

5. An autoclave according to Claim 4, characterised in that the control unit (4) only monitors the output of the air detection system (23) if the predetermined leak is within the predetermined limits.

6. An autoclave according to any one of the preceding claims, including a printer (31), and characterised in that signals from the control unit (4) are supplied to the printer (31) to provide a printed representation of autoclave function.

7. An autoclave according to any one of the preceding claims, characterised in that the control unit (4) is arranged to disable operation of the autoclave if the control unit detects a fault in the autoclave.
